# EUROPEAN PATENT SPECIFICATION

(11) **EP 3 013 258 B1**
(45) Date of publication and mention of the grant of the patent: **07.06.2017**
(21) Application number: 14742405.5
(22) Date of filing: 25.06.2014
(51) Int. Cl.: A61B 17/32, A61B 17/3207, A61M 27/00

(54) **CURETTE HEAD**
KÜRETTENKOPF
TÊTE DE CURETTE

(30) Priority: 26.06.2013 US 201361839479 P; 24.06.2014 US 201462016346 P
(43) Date of publication of application: 04.05.2016
(73) Proprietor: 3M Innovative Properties Company, St. Paul, MN 55133-3427 (US)
(72) Inventor: CASTRO, Gustavo H., Saint Paul, MN 55133-3427 (US); WILLOUGHBY, Jaime B., Saint Paul, MN 55133-3427 (US); ANDERSON, Gregory J., Saint Paul, MN 55133-3427 (US)
(74) Representative: Vossius & Partner Patentanwälte Rechtsanwälte mbB
(86) International application number: PCT/US2014/044086
(87) International publication number: WO 2014/210149

(56) References cited:
- WO-A1-96/32894
- WO-A1-2011/047140
- DE-C1- 10 138 235
- DE-U1- 20 110 351
- US-A1- 2004 030 254

## Description

### BACKGROUND

Debridement refers to the removal of dead, damaged, or infected tissue to improve the healing potential of the remaining healthy tissue. Several factors make proper debridement difficult such as poor lighting, difficult wound locations, immobile patients, environmental constraints, and the use of improper debridement tools. In addition, the action of cutting away the debrided eschar can be especially difficult because the blade, often a traditional pointed blade, may cut into healthy tissue and cause extensive bleeding. Therefore, this fear may cause some users to debride "lightly". This can often lead to infections due to eschar remaining in the wound after cleaning and redressing.

Current ulcer and wound care procedures and instruments fail to adequately address the need for proper ulcer and wound bed preparation by decreasing microbial colonization in the wound or ulcer other than using a medicated dressing or cream or ointment. They also fail to address the need for adequate removal of drainage in a wound or ulcer site that has become infected as a result of an infections process, or the creation of a nidus for infection due to the presence of residual cleaning solution that was not properly evacuated from the wound or ulcer site during the cleaning of the ulcer or wound.

US 2004/0030254 discloses a device and method for ultrasound wound debridement, wherein the device comprises a plurality of sharp protrusions extending from the base of a curette head. The device further comprising an elongate member through which a hollow passageway allows fluid communication for irrigation and suction purposes.

By addressing the problems associated with microbial colonization and infection prevention in pressure ulcers, surgically dehisced wounds, open wounds, or burns, a practitioner can substantially improve the bed state of an ulcer or wound by promoting a healing environment while significantly reducing the cost of treating these ulcers or wounds.

### SUMMARY

The disclosed embodiments provide solutions to problems associated with existing debridement tools. For example, in one embodiment, a debridement tool for debriding tissue from a tissue site is presented. The inventive curette head of the present disclosure provides a plurality of blades, including a first blade that delivers a fluid (e.g., a lubricating, rinsing, and/ or cleaning fluid) directly to the location of a wound site that is being debrided. Advantageously, this aspect of the invention can reduce the amount of fluid necessary to treat a wound during a debridement procedure. Optionally, the curette head comprises at least one additional blade that is adapted for removing the fluid and/or tissue debris from a wound site as the wound is being debrided. Advantageously, this aspect of the invention can permit better visualization of the wound site during the debridement process. In addition, the fluid is delivered, or removed, through openings in the blade that are located in or proximate the edge of the blade that is used for debriding the wound. Advantageously, this feature can minimize the volume of fluid needed for the debridement process and can minimize the volume of liquid "run-off" during the wound treatment, thereby keeping the treatment site clean and visible during the procedure. By incorporating liquid delivery and removal into a single device, the inventive apparatus combines multiple steps (e.g., rinsing and liquid removal) into a single process that requires only one hand for the operator to execute, leaving the other hand available to perform other procedures simultaneously.

In one aspect, the present disclosure provides an apparatus. The apparatus can comprise an elongate member having a first end and a second end, a curette head disposed at the first end, and a handle disposed at the second end. The curette head can include a base, a plurality of blades extending from the base, and at least one first hollow channel extending through the first blade. Each blade of the plurality of blades comprises a debridement edge and a longitudinal dimension extending from the base to the debridement edge. The debridement edge of each blade of the plurality of blades is defined by a first major surface and a second major surface of each respective blade of the plurality of blades. The plurality of blades can comprise a first blade having at least one first hollow channel extending therethrough. The at least one first hollow channel can extend along the first blade longitudinal dimension from a first opening in the base, or proximate the base, to at least one second opening that is integrated in the debridement edge of the first blade or that is disposed in the first major surface of the first blade proximate the debridement edge of the first blade. The handle can be attached to the curette head. The handle can comprise a first conduit extending therethrough, wherein the first conduit is in fluidic communication with the first hollow channel.

In another aspect, the present disclosure provides a curette head. The curette head can comprise a base, a plurality of blades extending from the base, and at least one first hollow channel extending through the first blade. Each blade of the plurality of blades can comprise a debridement edge and a longitudinal dimension extending from the base to the debridement edge. The debridement edge of each blade of the plurality of blades is defined by a first major surface and a second major surface of each respective blade of the plurality of blades. The plurality of blades can comprise a first blade. The at least one first hollow channel extends along the first blade longitudinal dimension from a first opening in the base, or proximate the base, to at least one second opening that is integrated in the debridement edge of the first blade or that is disposed in the first major surface of the first blade proximate the debridement edge of the first blade.

In any of the above embodiments, the plurality of blades further can comprise a second blade; wherein at least one second hollow channel extends through the second blade; wherein the at least one second hollow channel extends along the second blade longitudinal dimension from a third opening in the base, or proximate the base, to at least one fourth opening that is integrated in the debridement edge of the second blade or that is disposed in the first major surface of the second blade proximate the debridement edge of the second blade.

In any of the above embodiments, the plurality of blades further can comprise a third blade; wherein at least a portion of the second hollow channel extends through the third blade; wherein the portion of the second hollow channel extends along the third blade longitudinal dimension from an opening in the base, or proximate the base, to at least one fifth opening integrated in the debridement edge of the third blade or proximate the debridement edge of the third blade.

In yet another aspect, the present disclosure provides an exemplary method of treating a wound site. The method can comprise operatively connecting to a source of negative pressure the third opening, if present, of the curette head of any one of the above embodiments of the apparatus; placing at least two blades of the curette head of the apparatus into contact with tissue and/or tissue debris proximate the wound site; and while in contact with the tissue and/or tissue debris, moving the blades laterally relative to the wound site.

In yet another aspect, the present disclosure provides an exemplary method of treating a wound site. The method can comprise operatively connecting to a source of fluid the first opening of the curette head of any one of the above embodiments of the apparatus; placing at least two blades of the curette head of the apparatus into contact with tissue and/or tissue debris proximate the wound site; flowing the fluid through the at least one second opening; and, while in contact with the tissue and/or tissue debris, moving the blades laterally relative to the wound site.

In yet another aspect, the present disclosure provides a kit. The kit can comprise the curette head of any one of the above embodiments. Optionally, the kit further can comprise a handle configured to be operatively attached to the curette head.

In yet another aspect, the present disclosure provides an apparatus. The apparatus can comprise an elongate member having a first end and a second end, a curette head disposed at the first end, a body disposed at the second end, and a fluid-containing canister. The curette head can include a base and a plurality of blades extending from the base. Each blade of the plurality of blades can comprise a debridement edge and a longitudinal dimension extending from the base to the debridement edge. The debridement edge of each blade of the plurality of blades is defined by a first major surface and a second major surface of each respective blade of the plurality of blades. The plurality of blades comprises a first blade having at least one first hollow channel extending therethrough. The at least one first hollow channel can extend along the first blade longitudinal dimension from a first opening in the base, or proximate the base, to at least one second opening that is integrated in the debridement edge of the first blade or that is disposed in the first major surface of the first blade proximate the debridement edge of the first blade. The body can comprise a first conduit in fluid communication with the at least one first hollow channel. The fluid-containing canister can be in selective fluid communication with the first conduit.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

The terms "comprises" and variations thereof do not have a limiting meaning where these terms appear in the description and claims.

As used herein, "a," "an," "the," "at least one," and "one or more" are used interchangeably. Thus, for example, "a" blade can be interpreted to mean "one or more" blades.

The term "and/or" means one or all of the listed elements or a combination of any two or more of the listed elements.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

Additional details of these and other embodiments are set forth in the accompanying drawings and the description below. Other features, objects and advantages will become apparent from the description and drawings, and from the claims. The scope of the protection of the invention is defined by the appended claims.

### BRIEF DESCRIPTION OF DRAWINGS

FIG. 1 is a side view of one embodiment of an apparatus comprising an elongate member having one embodiment of a curette head and one embodiment of a handle according to the present disclosure.
FIG. 2 is a plan view of the front side of the curette head shown in FIG. 1.
FIG. 3 is a perspective view of the front side of the curette head shown in FIG. 1.
FIG. 4 is a cross-sectional view, along line 4-4, of the curette head shown in FIG. 2.
FIG. 5 is a partial side view of an alternative embodiment of a curette head according to the present disclosure.
FIG. 6 is a partial cross-sectional view of the curette head of FIG. 5.
FIG. 7 is a perspective view of the front side of the curette head of FIG. 5.
FIG. 8 is a perspective view of the front side of another alternative embodiment of a curette head according to the present disclosure.
FIG. 9 is a perspective view of yet another alternative embodiment of a curette head according to the present disclosure.
FIG. 10 is a perspective view of the back side of the curette head of FIG. 1.
FIG. 11 is a perspective view of a portion of the handle of the apparatus of FIG. 1, showing the portion that operably attaches to the curette head.
FIG. 12 is a plan view of the apparatus of FIG. 1, showing the front side of the curette head.
FIG. 13 is a plan view of the handle of FIG. 1, showing the back side of the curette head.
FIG. 14 is a cross-sectional view of the handle of FIG. 13 taken along the line 14-14.
FIG. 15 is a perspective view of the front side of yet another embodiment of a curette head according to the present disclosure.
FIG. 16 is a cross-sectional view of the curette head of FIG. 15.
FIG. 17 is a perspective view of the back side of the curette head of FIG. 15.
FIG. 18 is a perspective view of an apparatus comprising an elongate member having one embodiment of a curette head and an alternative embodiment of a handle according to the present disclosure.
FIG. 19 is a cross-sectional side view of the apparatus of FIG. 18.
FIG. 20 is a top view of the apparatus of FIG. 18.
FIG. 21 is a partially-exploded schematic block diagram of one end of an apparatus comprising a jet injector according to the present disclosure.

### DETAILED DESCRIPTION

In the following detailed description of several illustrative embodiments, reference is made to the accompanying drawings that form a part hereof, and in which is shown by way of illustration specific embodiments in which the invention may be practiced. These embodiments are described in sufficient detail to enable those skilled in the art to practice the invention, and it is understood that other embodiments may be utilized and that logical structural, mechanical, electrical, and chemical changes may be made without departing from the scope of the invention, as defined in the appended claims. To avoid detail not necessary to enable those skilled in the art to practice the embodiments described herein, the description may omit certain information known to those skilled in the art. The following detailed description is, therefore, not to be taken in a limiting sense, and the scope of the illustrative embodiments are defined only by the appended claims.

The terms "debride", "debriding", and "debridement" as used herein refers to the removal of necrotic tissue to improve the healing potential of the remaining healthy tissue. The term "necrotic tissue" as used herein refers to dead, damaged, or infected tissue. Although debridement of necrotic tissue may sometimes require an incision or cut be made, the disclosed embodiments may also be used to debride necrotic tissue without requiring that any incisions be made. The term "tissue site" as used herein refers to a wound or defect located on or within any tissue, including but not limited to, bone tissue, adipose tissue, muscle tissue, neural tissue, dermal tissue, vascular tissue, connective tissue, cartilage, tendons, or ligaments. The term "blade" as referenced herein refers to a component of the debridement tool that comes in contact with necrotic tissue and is capable of being used for debriding necrotic tissue from a tissue site. The term "blade" does not imply or infer that the blade is necessarily sharp or capable of cutting.

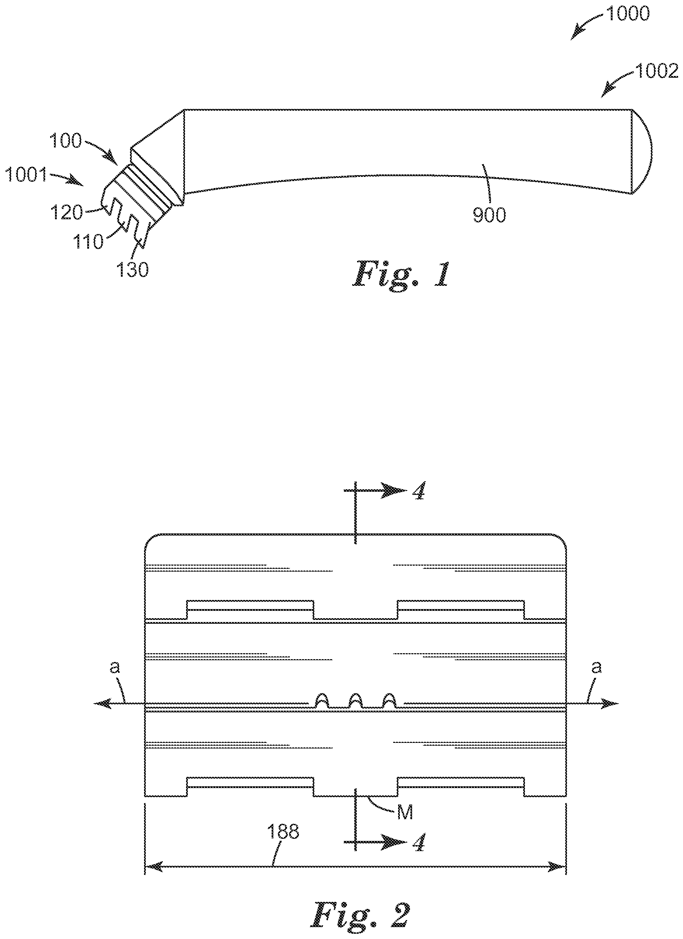

The present disclosure includes an inventive curette head that comprises a base and a plurality of blades extending from the base. In any embodiment, the curette head can be attached (e.g., detachably attached, such as attached via a quick-connect fitting) to a handle for use. Referring to the drawings, FIG. 1 shows one embodiment of an apparatus comprising an elongate member 1000. The elongate member 1000 comprises a curette head 100 at a first end 1001 of the elongate member attached to a handle 900 at a second end 1002 of the elongate member.

The curette head 100 (shown in FIGS. 2 through 3) includes a front side 198 and a back side 199. In use, the front side 198 of the curette head 100 is the patient-facing side. In any embodiment, the back side 199 can be configured to be operatively coupled to the handle 900.

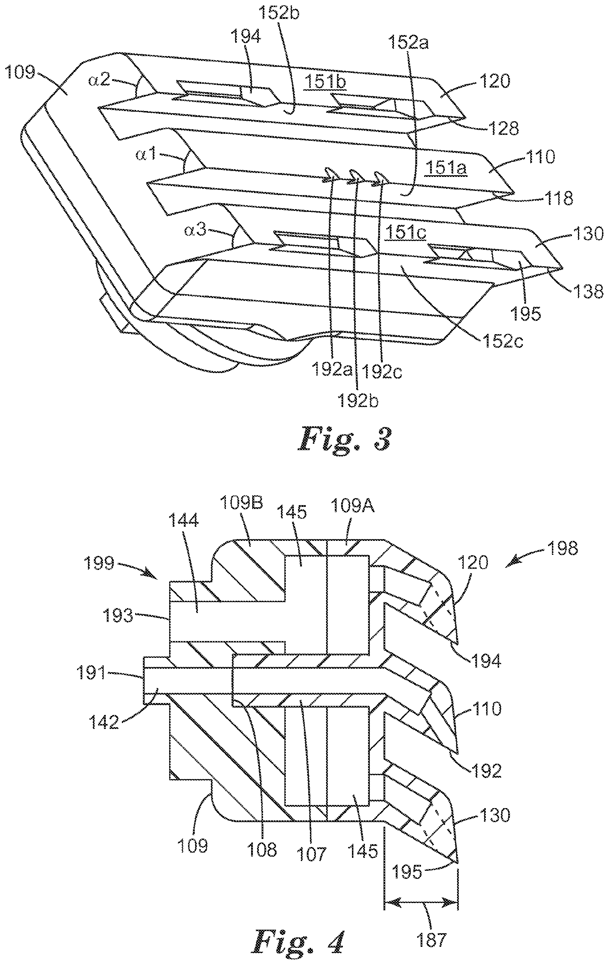

The front side 198 of the curette head 100 comprises a plurality of blades, including a first blade 110 and a second blade 120, extending from a base 109. In any embodiment, as illustrated in FIGS. 2-3, the plurality of blades further comprises an optional third blade 130. Each blade of the plurality of blades comprises a debridement edge (e.g., first debridement edge 118, second debridement edge 128, and third debridement edge 138, respectively). In addition, each blade of the plurality of blades comprises a longitudinal dimension (e.g., the longitudinal dimension of first blade 110, shown as line 187 in FIG. 2) extending from the base 109 to the respective debridement edge (e.g., first debridement edge 118) and a width dimension (e.g., the width dimension of first blade 110, shown as line 188 in FIG. 4) extending along debridement edge. The width dimension comprises a midpoint "M" that defines the center of the width dimension.

The debridement edge of each blade is formed at the junction of at least two major surfaces (e.g., the first debridement edge 118 is formed at the junction of first major surface 151a and second major surface 152a, the second debridement edge 128 is formed at the junction of first major surface 151b and second major surface 152b, and the third debridement edge 138 is formed at the junction of first major surface 151c and second major surface 152c). The angle (α) formed by the first and second major surfaces (151 and 152, respectively) is preferably less than or equal to 90°, more preferably, is less than 90° and, even more preferably, is about 5° to about 60°. In any embodiment, an angle α of less than or equal to 90° provides a debridement edge that is suitable for scraping or cutting a treatment surface of a wound to be debrided. In any embodiment, an angle of about less than or equal to about 30° can provide a debridement edge that is more suitable for cutting or slicing biological tissue or tissue debris at a wound site.

In a preferred embodiment, the angle α formed by the first and second major surfaces 151 and 152 of any one of the plurality of blades is about 5° to about 90°. In a more preferred embodiment, the angle α formed by the first and second major surfaces 151 and 152 of any one of the plurality of blades is about 5° to about 60°. In yet a more preferred embodiment, the angle α formed by the first and second major surfaces 151 and 152 of any one of the plurality of blades is about 5° to about 30°. Blade angles of about 5° or less can be suitable for cutting or slicing tissue and/or wound debris, whereas blade angles greater than 5° may be more suitable for abrading or scraping.

In any embodiment, the angle α formed by the first and second major surfaces and of the first blade is the same as the angle α2 formed by the first and second major surfaces of the second blade and/or the angle α3 formed by the first and second major surfaces of the third blade. In any embodiment, the angle α1 formed by the first and second major surfaces of the first blade is the different than the angle α2 formed by the first and second major surfaces of the second blade and/or the angle α3 formed by the first and second major surfaces of the third blade.

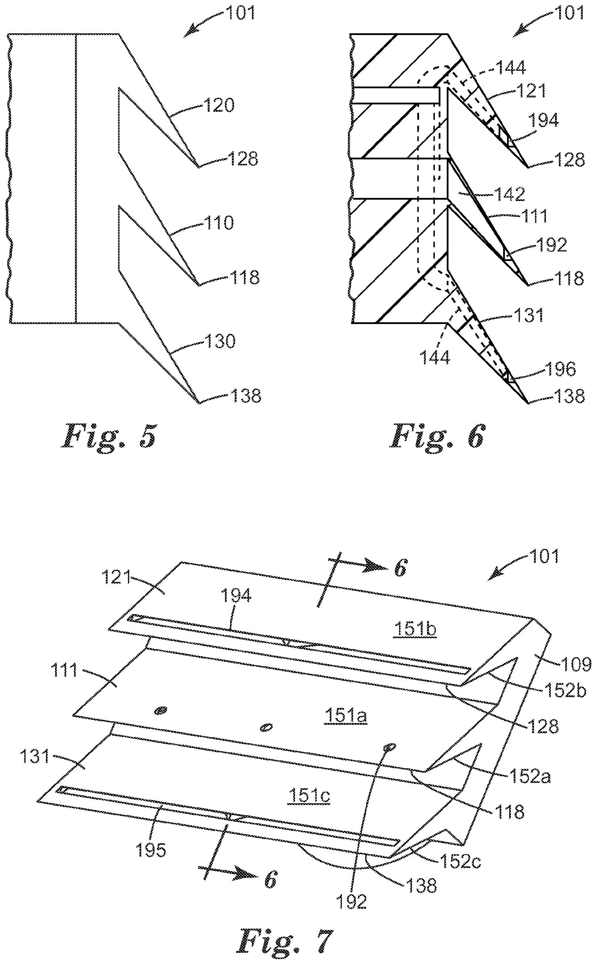

The curette head 100 comprises a first hollow channel 142 that extends from a first opening 191 disposed in the base 109 longitudinally through the first blade 110 to at least one second opening (e.g., second openings 192a, 192b, and 192c, respectively). In the illustrated embodiment, the at least one second opening is integrated in the first debridement edge 118. "Integrated in the debridement edge", as used herein, refers to an opening that extends along at least a portion of the debridement edge. In alternative embodiments shown in FIG. 7, for example, and described herein, the at least one second opening is disposed in the blade proximate, but not integrated in, the first debridement edge.

In any embodiment, the second openings comprise a minimum opening dimension (e.g., diameter). The minimum opening dimension preferably is about 10 µm to about 250 µm for openings that are used to deliver (or remove) liquids that have relatively low viscosity (e.g., deionized water, a buffered solution, a saline solution) and that are substantially free of particulate matter having a diameter of greater than about 1-5 µm. The minimum opening dimension preferably is about 250 µm to about 1000 µm for openings that are used to deliver (or remove) liquids that have relatively high viscosity (e.g., serous fluid) and/or that are contain particulate matter having a diameter of greater than about 10-500 µm.

In any embodiment, the first blade 110 can comprise a plurality of second openings 192. In any embodiment of a first blade 110 that comprises a plurality of second openings (e.g., second openings 192a, 192b, and 192c), the plurality of second openings form a distribution pattern. In any embodiment, the distribution pattern may consist essentially of a plurality of second openings that are biased toward the midpoint of the width dimension of the first blade (as shown in FIG. 3), or the distribution pattern may consist essentially of a plurality of second openings that are biased away from the midpoint of width dimension of the first blade (not shown). Advantageously, biasing the position of the plurality of second openings toward the midpoint can reduce the volume of liquid "run-off". In contrast, biasing the position of the plurality of second openings away from the midpoint can facilitate the distribution of liquid to the peripheral areas of the curette head. Alternatively, in any embodiment, the distribution pattern may comprise second openings that are approximately evenly spread across the width dimension of the first blade, as depicted in FIG. 7.

In any embodiment (not shown), at least one second opening of the plurality of second openings is disposed proximate the first debridement edge 118 and at least one second opening of the plurality of second openings is integrated in the first debridement edge.

In use, the first hollow channel 142 can be used to deliver a fluid to a treatment site or it can be used to remove debris from a treatment site. In any embodiment, the second openings 192 can be used to deliver (e.g., via positive pressure or gravity flow) a fluid (i.e., a gas or a liquid) directly to a treatment site as the curette head 100 contacts the treatment site. The fluid can be used to rinse or lubricate the plurality of blades in the curette head 100 and/or it can be used as a carrier fluid to suspend wound debris (e.g., in a liquid suspension) for easier removal. Advantageously, by delivering fluid (e.g., a rinsing fluid that is used to rinse the treatment site and the plurality of blades) to second openings in the first blade that are biased toward the midpoint of the width dimension, migration of the fluid away from the second openings spreads the fluid across the width of the blade, in the direction of arrows "a", thereby facilitating the rinsing and/or lubrication of the entire width of the blade.

Alternatively, in any embodiment, the second openings 192 can be used to remove (e.g., via negative pressure) a fluid and/or debris from a treatment site. The first hollow channel 142 can be operatively connected to a vacuum source and wound exudate and/or debris from the treatment site can be removed via the second openings 192.

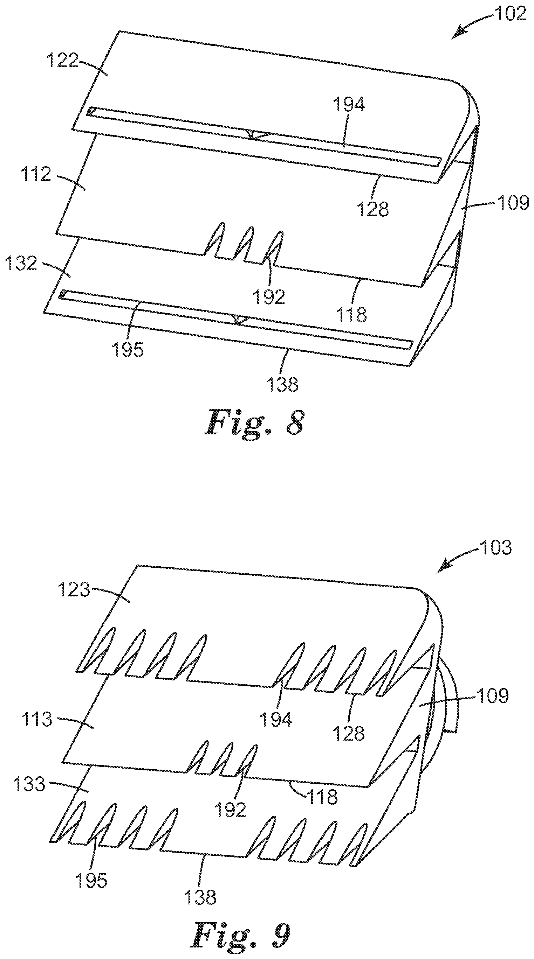

In any embodiment, the curette head optionally comprises a second hollow channel 144. The second hollow channel extends through the curette head 100 from the third opening 193 to the at least one fourth opening 194 and, optionally, the at least one fifth opening 195. The second hollow channel 144 comprises an optional chamber 145 into which fourth openings 194 and fifth openings 195 extend. In the illustrated embodiment, the at least one fourth opening is integrated in the second debridement edge 128. In any embodiment (as shown in FIGS. 7 and 8), the at least one fourth opening 194 can be disposed in the second blade 120 proximate, but not integrated in, the second debridement edge 128.

In any embodiment, the second blade 120 can comprise a plurality of fourth openings (e.g., fourth openings 194a and 194b). In any embodiment of a second blade 120 that comprises a plurality of fourth openings, the plurality of fourth openings form a distribution pattern. In any embodiment, the distribution pattern may comprise fourth openings that are approximately evenly spread across the width dimension of the second blade, as depicted in FIG. 7. Alternatively, in any embodiment, the distribution pattern may consist essentially of a plurality of fourth openings that are biased toward the midpoint of the width dimension of the second blade (not shown) or the distribution pattern may consist essentially of a plurality of fourth openings that are biased away from the midpoint of the width dimension of the second blade (see, for example, FIG. 9, discussed below). In an embodiment (not shown), at least one fourth opening of the plurality of fourth openings is disposed in the second blade 120 proximate the second debridement edge 128 and at least one fourth opening of the plurality of fourth openings is integrated in the second debridement edge 128.

In any embodiment, the fourth openings comprise a minimum opening dimension (e.g., diameter). The minimum opening dimension preferably is about 10 µm to about 250 µm for openings that are used to deliver (or remove) liquids that have relatively low viscosity (e.g., deionized water, a buffered solution, a saline solution) and that are substantially free of particulate matter having a diameter of greater than about 1-5 µm. The minimum opening dimension preferably is about 250 µm to about 1000 µm for openings that are used to deliver (or remove) liquids that have relatively high viscosity (e.g., serous fluid) and/or that are contain particulate matter having a diameter of greater than about 10-500 µm.

In any embodiment, the curette head 100 optionally comprises a third blade 130. The third blade 130 optionally is in fluid communication with the second hollow channel 144 and at least a portion of the second hollow channel extends longitudinally through the third blade 130 to at least one fifth opening (e.g., fifth openings 195a and 195b, respectively). In the illustrated embodiment of FIG. 3, the at least one fifth opening 195 is disposed in the third blade 130 proximate the third debridement edge 138.

In any embodiment, the third blade 130 can comprise a plurality of fifth openings (e.g., fifth openings 195a and 195b). In any embodiment of a third blade 130 that comprises a plurality of fifth openings, the plurality of fifth openings forms a distribution pattern. In any embodiment, the distribution pattern may comprise fifth openings that are approximately evenly spread across the width dimension of the third blade, as depicted in FIGS. 7 and 8. Alternatively, in any embodiment, the distribution pattern may consist essentially of a plurality of fifth openings that are biased toward the midpoint of the width dimension of the third blade (not shown) or the distribution pattern may consist essentially of a plurality of fifth openings that are biased away from the midpoint of the width dimension of the third blade (see, for example, FIG. 9, discussed below). In an embodiment (not shown), at least one fifth opening of the plurality of fifth openings is disposed in the third blade 130 proximate the third debridement edge 138 and at least one fifth opening of the plurality of fifth openings is integrated in the third debridement edge.

In any embodiment, the fifth openings comprise a minimum opening dimension (e.g., diameter). The minimum opening dimension preferably is about 10 µm to about 250 µm for openings that are used to deliver (or remove) liquids that have relatively low viscosity (e.g., deionized water, a buffered solution, a saline solution) and that are substantially free of particulate matter having a diameter of greater than about 1-5 µm. The minimum opening dimension preferably is about 250 µm to about 1000 µm for openings that are used to deliver (or remove) liquids that have relatively high viscosity (e.g., serous fluid) and/or that are contain particulate matter having a diameter of greater than about 10-500 µm.

In the illustrated embodiment of FIGS. 2-4, the base of the curette head 100 comprises two portions (base portions 109A and 109B, respectively) that are press-fit together via the extension structure 107 in base portion 109A that is configured to be received by and to be coupled with cavity 108 in base portion 109B. In addition, the base portions 109A and 109B can be sealed together (e.g., by an adhesive (not shown), or other means known in the art, in order to form prevent leakage.

In use, the first hollow channel 142 can be used to deliver a fluid to a treatment site or it can be used to remove debris from a treatment site. In a preferred embodiment, the second openings 192 are used to deliver (e.g., via positive pressure or gravity flow) a fluid (i.e., a gas or a liquid), such as an liquid containing a medicament or antimicrobial, directly to a treatment site as the curette head 100 contacts the treatment site and the fourth and/or fifth openings (openings 194 and 195, respectively) are operatively connected to a source of negative pressure, thereby permitting the fluid and/or tissue debris to be drawn away from the treatment site through the second hollow channel 144. The configuration of the second openings 192, fourth openings 194 and fifth openings 195 shown in FIGS. 3 and 4; wherein the second openings 192 are biased toward the midpoint of the first debridement edge 118 and the fourth openings 194 and fifth openings 195 extend away from the midpoint of the second debridement edge 128 and third debridement edge 138, respectively; is particularly well-suited to dispense a liquid through the second openings 192 and collect the liquid (and any loose tissue debris, if present) via the fourth openings 194 and/or fifth openings 195.

In any embodiment, the debridement edge of at least one of the plurality of blades may form a sharp, knife-like edge that is configured for scraping (e.g., with minimal cutting) biological matter (e.g., tissue and/or biological matter derived therefrom). In any embodiment, the debridement edge of at least one of the plurality of blades may form an edge that is configured for removing biological matter (e.g., tissue and/or biological matter derived therefrom) from a treatment site without cutting the matter (e.g., removing it by wiping or scraping). It is therefore contemplated that, in any embodiment, the debridement edge of at least one of the plurality of blades may be relatively dull (e.g., the debridement edge is rounded).

In a preferred embodiment of the curette head of the present disclosure, the first blade is disposed on the front side of the curette head and between a second blade and a third blade, as shown in the illustrated embodiments.

FIGS. 5-7 show various views of an alternative embodiment of a curette head 101 according to the present disclosure. The curette head 101 comprises a first blade 111 comprising a plurality of second openings 192 that are substantially evenly distributed across the width dimension of the first blade 111. The first debridement edge 118 is formed at the junction of a first major surface 151a and a second major surface 152a of the first blade 111. Each second opening 192 is disposed in the first major surface 151a of the first blade 111 proximate the first debridement edge 118. In an alternative embodiment (not shown), at least one second opening 192 is disposed in the second major surface 152a of the first blade 111 proximate the first debridement edge 118. Similar to the curette head 100 shown in FIGS. 2-4, the second openings 192 in the curette head 101 are in fluid communication with a first opening (not shown in FIGS.5-7), located on the back side or proximate the back side of the curette head 101, via a portion of the first hollow channel 142.

The curette head 101 further comprises a second blade 121 comprising a plurality of fourth openings 194 that optionally extend across approximately the entire width dimension of the second blade 121. The second debridement edge 128 is formed at the junction of a first major surface 151b and a second major surface 152b of the second blade 121. Each fourth opening 194 is disposed in the second blade 121 proximate the second debridement edge 128. In an alternative embodiment (not shown), at least one fourth opening 194 is disposed in the second major surface 152b of the second blade 121 proximate the second debridement edge 128. Similar to the curette head 100 shown in FIGS. 2-4, the fourth openings 194 in the curette head 101 are in fluid communication with a third opening (not shown in FIGS. 5-7) - located on the back side or proximate the back side of the curette head 101 - via the second hollow channel 144.

In any embodiment, the fourth openings 194 can be used to deliver (e.g., via positive pressure or gravity flow) a fluid (i.e., a gas or a liquid) to a treatment site. Alternatively, in any embodiment, the fourth openings 194 can be used to remove (e.g., via negative pressure) a fluid and/or debris from a treatment site. Advantageously, by removing the fluid (e.g., a rinsing fluid that is used to rinse the treatment site and the plurality of blades) using fourth openings extending substantially across the entire width of the second blade, a treatment area defined by the path of the curette head during use can be kept substantially free of residual fluid and/or tissue debris.

The curette head 101 further comprises a third blade 131 comprising a plurality of fifth openings 195 that optionally extend across approximately the entire width dimension of the third blade 131. The third debridement edge 138 is formed at the junction of a first major surface 151c and a second major surface 152c of the third blade 131. Each fifth opening 195 is disposed in the third blade 130 proximate the third debridement edge 138. In an alternative embodiment (not shown), at least one fifth opening 195 is disposed in the first major surface 151c of the third blade 131 proximate the third debridement edge 138. Similar to the curette head 100 shown in FIGS. 2-4, the fifth openings 195 in the curette head 101 are in fluid communication with a third opening (not shown in FIGS. 5-7), located on the back side or proximate the back side of the curette head 101, via a portion of the second hollow channel 144.

In any embodiment, the fifth openings 195 can be used to deliver (e.g., via positive pressure or gravity flow) a fluid (i.e., a gas or a liquid) to a treatment site. Alternatively, in any embodiment, the fifth openings 195 can be used to remove (e.g., via negative pressure) a fluid and/or debris from a treatment site. Advantageously, by removing the fluid (e.g., a rinsing fluid that is used to rinse the treatment site and the plurality of blades) using fifth openings that extend substantially across the entire width of the third blade, a treatment area defined by the path of the curette head during use can be kept substantially free of residual fluid and/or tissue debris.

FIG. 8 shows a perspective view of the front side 198 of yet an alternative embodiment of a curette head 102 according to the present disclosure. The curette head 102 comprises a first blade 112 comprising a plurality of second openings 192 that are biased toward the midpoint of the width dimension of the first blade 112. Each second opening 192 is integrated in the first debridement edge 118. Similar to the curette head 100 shown in FIG. 5, the second openings 192 in the curette head 102 are in fluid communication with a first opening (not shown in FIG. 8), located on the back side or proximate the back side of the curette head 102, via a first hollow channel (not shown).

The curette head 102 further comprises a second blade 122 comprising a plurality of fourth openings 194 that extend substantially across the entire width dimension of the second blade 122. Each fourth opening 194 is integrated in the second debridement edge 128. Similar to the curette head 100 shown in FIGS. 2-4, the fourth openings 194 in the curette head 102 are in fluid communication with a third opening (not shown in FIG. 8), located on the back side or proximate the back side of the curette head 102, via a portion of a second hollow channel (not shown).

The curette head 102 further comprises a third blade 132 comprising a plurality of fifth openings 195 that extend substantially across the entire width dimension of the third blade 132. Each fifth opening 195 is integrated in the third debridement edge 138. Similar to the curette head 100 shown in FIG. 5, the fifth openings 195 in the curette head 102 are in fluid communication with a third opening (not shown in FIG. 8), located on the back side or proximate the back side of the curette head 102, via a portion of a second hollow channel (not shown).

FIG. 9 shows a perspective view of yet another curette head 103 according to the present disclosure. The curette head 103 comprises a first blade 113 comprising a plurality of second openings 192 that are biased toward the midpoint of the width dimension of the first blade 113. Each second opening 192 is integrated in the first debridement edge 118. Similar to the curette head 100 shown in FIG. 5, the second openings 192 in the curette head 103 are in fluid communication with a first opening (not shown in FIG. 8), located on the back side or proximate the back side of the curette head 103, via a first hollow channel (not shown).

The curette head 103 further comprises a second blade 123 comprising a plurality of fourth openings 194 that are biased away from the midpoint of the width dimension of the second blade 122. Each fourth opening 194 is integrated in the second debridement edge 128. Similar to the curette head 100 shown in FIGS. 2-4, the fourth openings 194 in the curette head 103 are in fluid communication with a third opening (not shown in FIG. 8), located on the back side or proximate the back side of the curette head 103, via a portion of a second hollow channel (not shown).

The curette head 103 further comprises a third blade 133 comprising a plurality of fifth openings 195 that are biased away from the midpoint of the width dimension of the third blade 133. Each fifth opening 195 is integrated in the third debridement edge 138. Similar to the curette head 100 shown in FIG. 5, the fifth openings 195 in the curette head 103 are in fluid communication with a third opening (not shown in FIG. 8), located on the back side or proximate the back side of the curette head 103, via a portion of a second hollow channel (not shown).

In another aspect, the present disclosure provides an apparatus. The apparatus can be used, for example, to treat a wound site (e.g., a surgical wound, burned tissue, a chronic wound, a pressure sore). The apparatus comprises an elongate member as described herein.

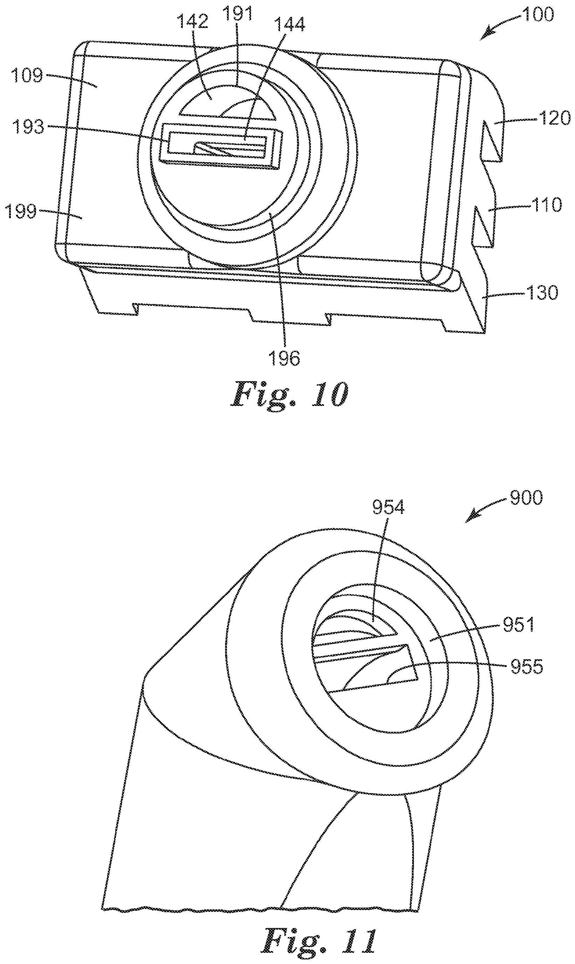

FIG. 10 shows a perspective view of the back side 195 of the curette head 100 of FIGS. 2-4. In any embodiment, the back side 195 comprises a raised feature 196 configured to attach (e.g., slideably engage) to a complementary-shaped structure in the handle (e.g., cavity 951 of the handle 900, shown in FIG. 11). Preferably, the raised feature 196 engages with the cavity 951 (e.g., via friction fit) to form a fluid-tight seal. The raised feature 196 preferably provides a fluid-tight connection between the curette head 100 and the handle 900. Also shown in FIG. 10 is the first opening 191, which is configured to fluidically couple with the first conduit 954 (FIG. 11) in the handle 900. Extending from the raised feature 196 on the back side 195 of the curette head 100 is a flange 197. When the curette head 100 is operatively coupled to the handle 900 of FIG. 11, the flange 197 extends into the second conduit 955 of the handle 900. In any embodiment, the back side further comprises a third opening 193. It is contemplated that a person having ordinary skill in the art will recognize other means by which a curette head of the present disclosure can be fluidically coupled to a handle to form the elongate member of the present disclosure.

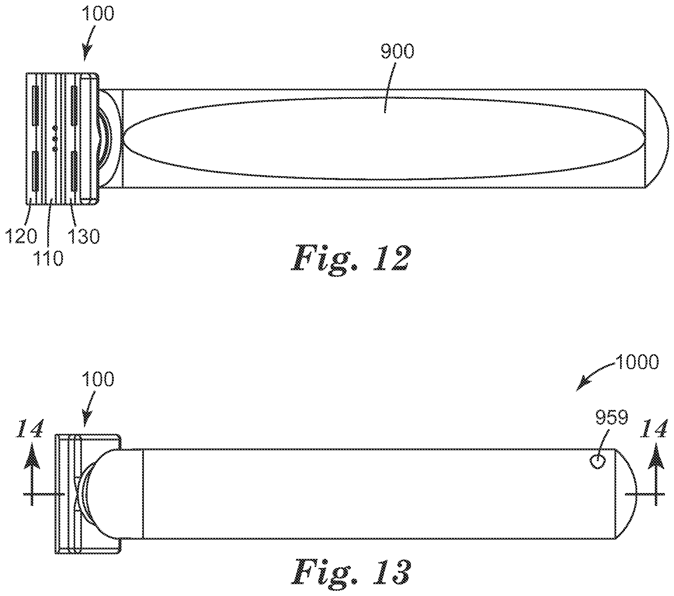

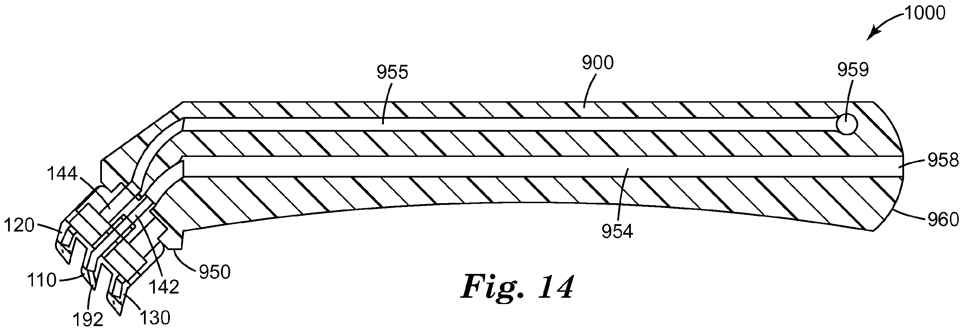

FIGS. 12-14 show various views of one embodiment of an apparatus comprising the assembled elongate member 1000 of the present disclosure. The elongate member 1000 comprises a curette head 100 and a handle 900. The curette head comprises a base with a plurality of blades (e.g., blades 110, 120, and 130, respectively) extending therefrom. Each blade of the plurality of blades comprises a debridement edge and a longitudinal dimension extending from the base to the debridement edge, as described hereinabove. At least one blade of the plurality of blades comprises a hollow channel extending longitudinally therethrough from a first opening in the base or proximate the base to at least one second opening that is integrated in the debridement edge or is disposed in a major surface of the blade proximate the debridement edge, as described hereinabove.

The handle 900 comprises a first end 950 and a second end 960. The first end 950 is adapted to be attached (e.g., permanently attached or detachably attached) to any embodiment of the curette head disclosed herein. Extending through at least a portion of the handle 900 are the first conduit 954 and the second conduit 955.

The first conduit 954 comprises a first coupling element 958 proximate the second end 960 of the handle 900. The first coupling element 958 is configured to be coupled to a fluid source (e.g., a liquid source). In any embodiment, the first coupling element 958 may be, for example, a female-type cavity that is shaped and dimensioned to receive a complementary-shaped male coupling element (not shown). In an alternative embodiment (not shown), the first coupling element may be a male-type projection that is shaped and dimensioned to be inserted into a complementary-shaped female-type coupling element. The first conduit 954 can deliver a fluid (e.g., a cleaning, lubricating, and/or rinsing liquid) to a wound treatment site via the first hollow channel 142 and the second opening 192 of the curette head 100. In any embodiment, the fluid may comprise an active agent (e.g., a medicament, a foaming agent). In any embodiment, the fluid may be a pressurized fluid.

In any embodiment of the elongate member, the second end 960 of the handle 900 may comprise may comprise a canister support (described below) proximate the coupling element 958 and the coupling element 958 may comprise a valve seat (described below). In these embodiments (not shown), the handle is configured to receive a portable source of fluid (e.g., a fluid-filled canister) as described herein.

The second conduit 955 comprises a second coupling element 959 proximate the second end of the handle. The second coupling element 959 is configured to be coupled to a fluid source (e.g., a liquid source) or a source of negative pressure (vacuum). In any embodiment, the second coupling element 959 may be, for example, a female-type cavity that is shaped and dimensioned to receive a complementary-shaped male coupling element (not shown). In an alternative embodiment (not shown), the second coupling element may be a male-type projection that is shaped and dimensioned to be inserted into a complementary-shaped female-type coupling element. The second conduit 955 can be used to remove (e.g., via negative pressure) tissue exudate, tissue debris, and/or a fluid delivered to the wound treatment site via the first hollow channel 142.

In any embodiment, an apparatus of the present disclosure further comprises a reservoir (not shown). In any embodiment (not shown), the handle may comprise the reservoir. In any embodiment (not shown), the reservoir may comprise a container (e.g., a cartridge, a pressurized cartridge) that can be operatively attached to the first hollow channel, second hollow channel, first conduit, or second conduit in order to deliver fluid from the reservoir to one or more second opening, fourth opening or fifth opening. The reservoir may comprise a valve (not shown). The valve may be actuated by an actuator (e.g., a Lorentz force actuator, not shown, as discussed herein).

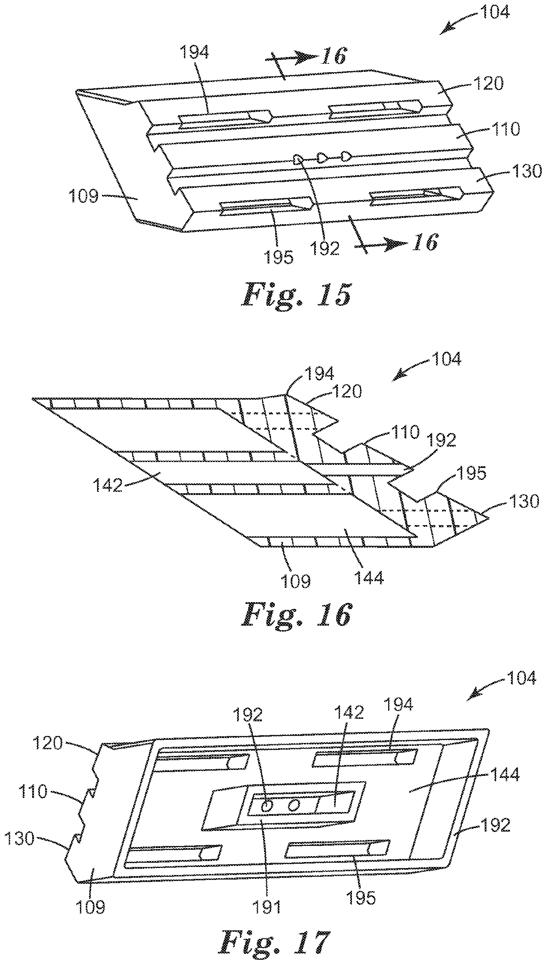

FIGS. 15-17 show yet another embodiment of a curette head 104 according to the present disclosure. The curette head 104 comprises a unitary body (i.e., the curette head is formed as a single part) having a base 109 with a plurality of blades (i.e., first blade 110, second blade 120, and third blade 130) extending therefrom. The first blade 110 comprises a plurality of second openings 192 and is in fluidic communication with a first hollow channel 142 formed in the unitary body. The first hollow channel 142 can be operatively connected to a source of fluid (not shown) or a source of negative pressure (not shown). The second blade 120 comprises a plurality of fourth openings 194 and is in fluidic communication with a second hollow channel 144 formed in the unitary body. The third blade 130 comprises a plurality of fifth openings 195 and is in fluidic communication with a second hollow channel 144 formed in the unitary body. The second hollow channel 144 can be operatively connected to a source of negative pressure (not shown), as described above for other embodiments of the curette head. The base 109 is configured to be operatively attached (e.g., by friction fit, sonic weld, heat bond, pressure sensitive adhesive, or the like) to a handle (not shown) that comprises a first conduit and/or second conduit as described above for other embodiments of the handle.

Although all of the illustrated embodiments of a curette head of the present disclosure comprise three blades, it is contemplated that the curette head may comprise only one blade. In the single-blade embodiment (not shown), the curette head comprises a base with the blade extending therefrom. The blade comprises a debridement edge, a longitudinal dimension extending from the base to the debridement edge, at least one hollow channel extending along the longitudinal dimension from a first opening in the base, or proximate the base, to at least one second opening that is integrated in the debridement edge of the blade or that is disposed in a first major surface of the first blade proximate the debridement edge of the first blade. The single-blade curette head can be operatively connected to a handle, a source of fluid, and/or a source of negative pressure as described herein.

In any embodiment of the curette head or apparatus described herein, it is contemplated that two or more of the openings (in one blade or in separate blades) can be connected to separate sources of fluids (not shown). This arrangement could permit simultaneous or sequential application of two separate liquids (e.g., a liquid comprising a medicament or cleanser can be applied through one opening and a rinse solution can be applied through another opening). In addition, it is contemplated that any opening can be fluidically connected to two or more sources of fluids (not shown). This arrangement could permit simultaneous or sequential application of two separate liquids through the same opening. The control of the separate liquids could be maintained using valve mechanisms that are known in the art.

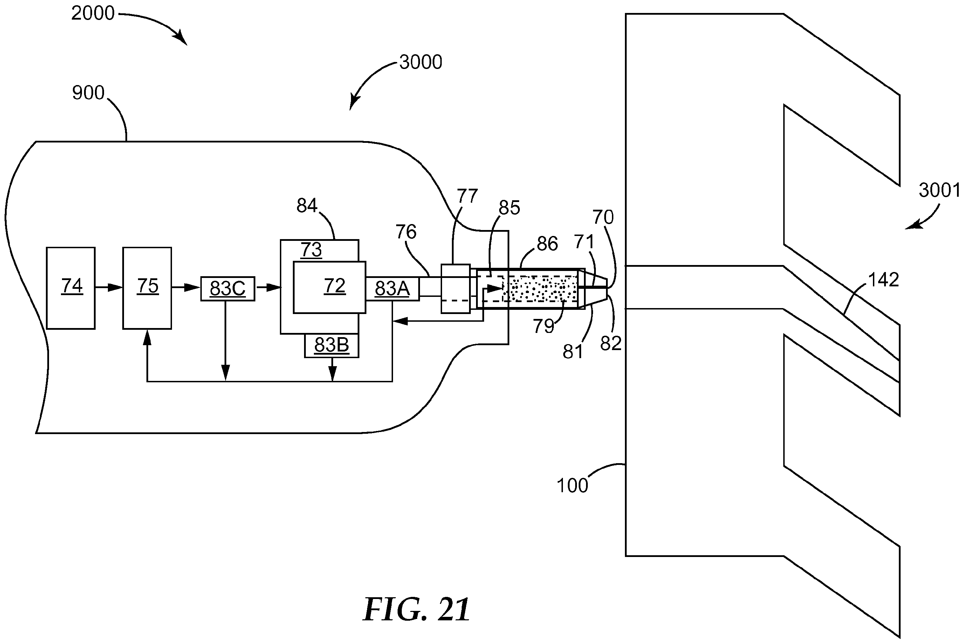

In any embodiment, the apparatus or curette head of the present disclosure may comprise or be operatively coupled to a jet injector (see FIG. 21). The jet injector can be used to deliver a liquid to the curette head. In any embodiment, the jet injector may be actuated by an actuator. In any embodiment, the actuator may comprise a Lorentz force actuator as described herein.

In any embodiment (not shown), the curette head may comprise a vibratory element or may be operatively connected to a vibratory element. The vibratory element preferably generates vibration (e.g., low-frequency ultrasonic vibration) that is transmitted through the curette head to the treatment site. Advantageously, the vibratory energy can dislodge and/or break apart wound debris, thereby facilitating removal of the wound debris from the treatment site. The use of vibratory energy in tissue therapy, surgical, and dental procedures is known in the art (see, for example, Ultrasonics: Fundamentals, Technology, Applications"; Dale Ensminger, Ed.; pp 530-551; Marcel Dekker, Inc., New York, NY).

In any embodiment of a curette head of the present disclosure, the debridement edges of the plurality of blades are spaced apart (i.e., the debridement edges are not contiguous). In any embodiment, the debridement edge of the first blade is substantially parallel to the debridement edge of the second blade. In any embodiment, the debridement edge of the second blade is substantially parallel to the debridement edge of the third blade. In any embodiment, the debridement edge of the first blade is substantially parallel to the debridement edge of the third blade. In any embodiment, the debridement edges of all of the blades in the plurality of blades are substantially parallel to each other. In any embodiment, the first, second, and third blades are stacked. In any embodiment, in the arrangement of stacked blades, the first blade is disposed between the second blade and the third blade, as shown in the illustrated embodiments shown and described herein.

In any embodiment, curette heads of the present disclosure can be fabricated using injection-molding processes known in the art. The mold part that forms the front side (i.e., including the plurality of blades) can be formed by using a silicon insert molding process (see, for example, J.B. Werkmeister ("Development of Silicon Insert Molded Plastic (SIMP)"; Thesis (Mech. E.); 2005; Massachusetts Institute of Technology; Cambridge, MA).

In the silicon insert molding process, inserts are placed inside a mold cavity. The silicon inserts are manufactured using microfabrication techniques allowing for tolerances, smoothness, and dimensions at a nanoscale level. For example, anisotropic etching is used to preferentially etch certain planes of a crystal lattice at a greater rate. A common etchant (e.g., potassium hydroxide) is used to remove selected planes of silicon faster than other planes, resulting in a nanometer smooth plane having a predetermined angle that is used in a mold to form curette head blades of the present disclosure. The angle can be varied by cutting an off-axis wafer from the ingot at an angle other than 90 degrees. Since the silicon inserts have atomically smooth surfaces, the molded parts can have dimensions on the micron scale with tolerances in the nanometer to micron range. The etched silicon structure (e.g., wafer) can then be interfaced to a traditional mold (e.g., a mold defining features of the curette head other than the blades). The traditional mold can be made using traditional machining operations and electro-discharge machining (EDM). By constraining the precise features (e.g., the blades and blade edges) within the silicon insert, the resulting curette heads can easily have highly precise regions (i.e., the blades and blades and blade edges) and less precise regions (e.g., the base from which the blades project).

In any embodiment, silicon wafers can be bonded together to make the mold insert that is used to form at least a portion (e.g., the blade portion) of the curette heads of the present disclosure. The wafers can be bonded together by anodic bonding or fusion bonding. Anodic bonding can be performed, for example, by placing the materials (e.g., a silicon wafer and a glass substrate) between two metal electrodes. After heating them to ∼400°C, a DC potential (e.g., up to 1kV) is applied to the top wafer. The fusion bonding process generally requires that the two substrates to be fused are heated (e.g., heated to 600 °C for about 4 - 6 hours). The heat treatment creates a fusion bond between the two wafers.

In addition, core pins can be inserted through the silicon wafer in order to form the hollow channels in each blade. The use of core pins to manufacture a molded object (e.g., a hollow microneedle) is described in International Publication No. WO 2010/11 7602. Typically, the mold comprises two parts (e.g., one part that forms the front side of the curette head and another part that forms the back side of the curette head. Bringing a core pin (e.g., a core pin disposed in a first mold part) into close proximity with a wall (e.g., a wall disposed in a second mold part) can result in a through-hole extending through an article that is molded using the first and second mold parts, as shown for example in FIGS. 4A-B through 9A-C, of International Publication No. WO 2010/117602.

A person having ordinary skill in the art will recognize that, in addition to injection molding, other processes may be used to manufacture the blades and/or curette head of the present disclosure. For example, an additive process, such as stereolithography, can be used to make the blades, curette heads, and/or handles. Alternatively, the blades, curette heads, and/or handles can be made using a 3-D printing process. In addition, an overmolding process can be used to produce a curette head having a plurality of blades with different durometers (i.e., one or more blades relatively softer than at least one other blade).

The curette heads of the present disclosure preferably are fabricated (e.g., by injection molding) using a polymeric thermoplastic material. Nonlimiting examples of suitable thermoplastic polymers that can be used to make the curette heads include polyolefins, styrenics, vinyls, acrylics, fluoropolymers, polyesters, polyamides, polyethers, and polyurethanes. Preferred materials for use in fabricating the curette heads of the present disclosure include polycarbonate, liquid crystal polymer, and polyurethane. When using an overmolding process, thermoplastic elastomers may be used.

It is contemplated that, in any embodiment, the apparatus or the curette head of the present disclosure can be disposable (i.e., intended for single-use). It is further contemplated that the apparatus and/or curette head can be disinfected or sterilized using methods that are well known in the art (e.g., steam sterilization, ethylene oxide sterilization, gamma irradiation).

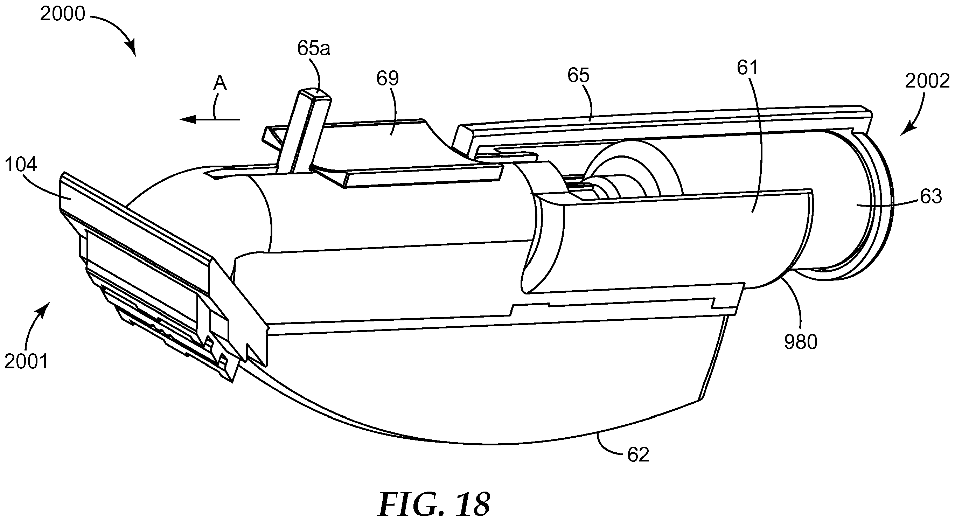

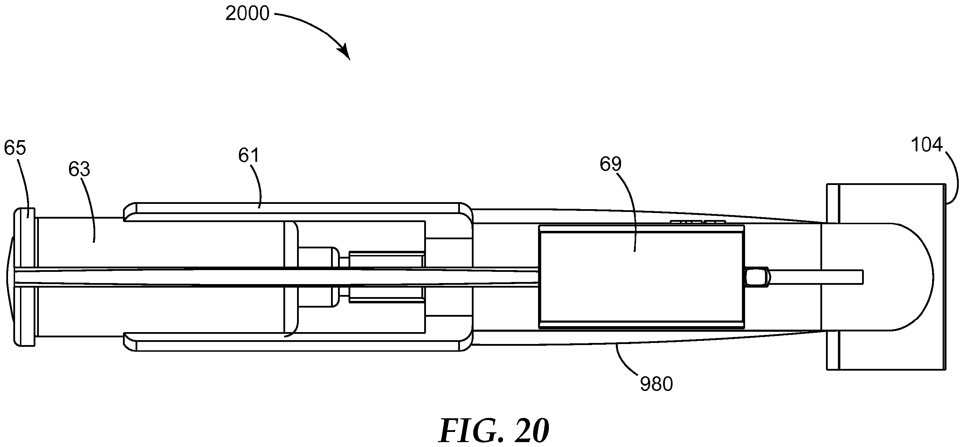

Referring back to the drawings, FIGS. 18-20 show various views of an alternative embodiment of an apparatus 2000 according to the present disclosure. The apparatus 2000 can be used as a debridement tool in a process of debriding a wound, as described hereinabove. The apparatus 2000 comprises a curette head (e.g., the curette head 104 as described herein) at a first end 2001 of the elongate member. The curette head 104 is attached to a body 980 that extends to a second end 2002 of the apparatus 2000. The body 980 can serve as a handle for grasping the apparatus 2000 during use. The body 980 can be fabricated using similar processes and materials as those used to fabricate the handle (e.g., handle 900 of FIG. 1) described hereinabove.

In the illustrated embodiment of FIGS. 18-20, the body 980 includes a number of features that permit the operator to use the apparatus (e.g., in a debridement procedure) without having to connect the apparatus to an auxiliary source of negative pressure (e.g., a vacuum line) and/or to a line (e.g., a hose, pipe, or tubing) that provides a wash liquid.

In any embodiment, the body 980 further includes a canister support 60 that is configured to receive and support a canister 63 during use. In addition, the body 980 can comprise an optional valve seat 68. The body 980 includes a first conduit 954 that extends from the valve seat 68 to the curette head (e.g., curette head 104). The first conduit 954 is in fluid communication with the second openings 192 (see FIG. 17) of the curette head 104. Thus, a fluid introduced into the first conduit 954 (e.g., from the canister 63) at the valve seat 68 can flow to and be discharged from one or more openings in the curette head 104.

In any embodiment, the canister can be a typical pressure canister (e.g., a metal canister designed to hold fluid contents under pressure) known in the art. In any embodiment, the canister 63 contains a gas and/or a liquid (e.g., deionized water, a buffered solution, a saline solution; not shown) under pressure. In any embodiment, the liquid may be a sterile liquid. In any embodiment, the canister 63 comprises a canister valve 67 configured to selectively release contents of the canister. Thus, the canister valve 67, when operatively positioned proximate the first conduit 954 (e.g., at the valve seat 68), places the canister in selective fluid communication with the first conduit. The canister valve 67 may be a spring-biased type valve known in the art wherein, for example, when the valve 67 is urged against the valve-seat 68, the valve 67 opens to release the contents of the canister 63. Preferably, the canister 63 is pressurized so that, when the canister valve 67 is actuated, fluid form the canister 63 is delivered through the conduit 954 through the curette head 104 to the wound (not shown) that is being treated with the apparatus.

In any embodiment, the body 980 can include an elastically-deformable wall 62 that at least partially defines a chamber 64 disposed in the body 980. The elastically-deformable wall 62 can be fabricated from a material (e.g., butyl rubber) that deforms (e.g., toward the body 980), for example, under manual pressure. This deformation of the wall 62 allows contents (e.g. air) in the chamber 64 to be expelled (e.g., through the curette head) from the chamber. In any embodiment, when pressure against the deformable wall 62 is released, the wall can elastically rebound to its previous state. Additionally, or alternatively, the apparatus may comprise a biasing element (e.g., a spring, not shown) that urges the elastically-deformable wall 62 substantially back to its previous condition. The movement of the elastically-deformable wall 62 back toward its previous condition creates a negative pressure within the chamber 64.

The chamber 64 is in fluid communication with at least one fourth opening 194 and/or fifth opening 195 of the curette head 104 (see FIG. 17) via the second conduit 955. Thus, when positive pressure is created within the chamber 64, any material that is partially or completely blocking the at least one fourth opening 194 and/or fifth opening 195 of the curette head 104 may be forced away from the opening by the positive pressure. Conversely, when negative pressure is created within the chamber 64, any material in or proximate the at least one fourth opening 194 and/or fifth opening 195 of the curette head 104 can be drawn through the curette head 104 into the chamber 64 via the first conduit 954.

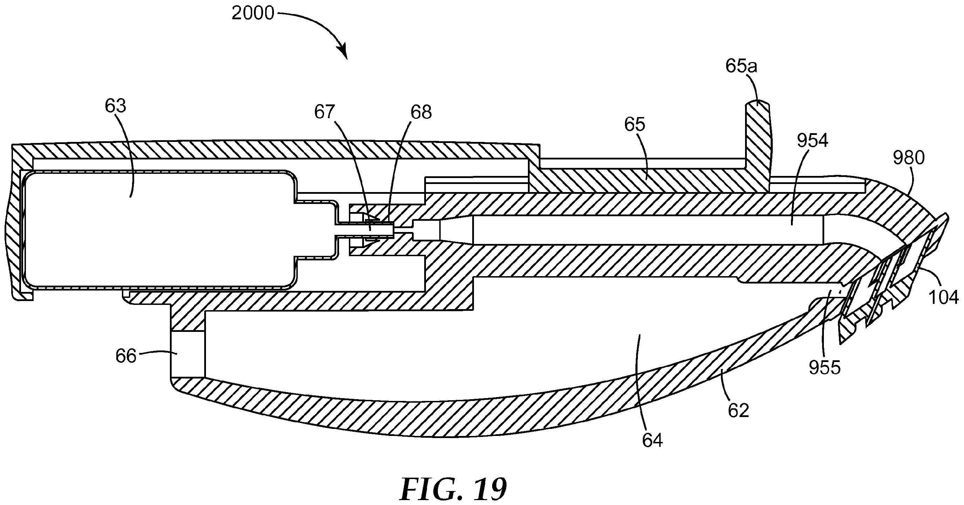

Also shown in FIG. 19 is an optional one-way valve 66. The valve 66, when present, allows fluid (e.g., air) to exit the chamber 64 but does not allow fluid to enter the chamber. In any embodiment, the one-way valve 66 can have an opening that is larger than the at least one fourth opening 194 and/or fifth opening 195 of the curette head 104. In these embodiments, when the elastically-deformable wall 62 is deformed, the contents (e.g., air) in the chamber 64 are preferentially expelled through the valve 66, thereby facilitating rapid evacuation of the contents of the chamber.

In any embodiment, the canister 63 can be urged against the valve seat 68 manually. Optionally, the elongated member 2000 comprises an actuator 65. The actuator 65 operatively engages the canister 63 such that, when a portion 65a of the actuator is moved (e.g., by finger pressure, in the direction of arrow 78) from a first position to a second position, the canister 63 is urged toward the body 980, thereby urging the canister valve 67 against the valve seat 68 causing actuation of the valve 67.

In any embodiment, the body 980 further comprises a platform 69. The platform 69 can be used to position a finger (e.g., a thumb, not shown) in an appropriate location to hold the elongated member 2000 and to align the finger with the portion 76 that is used to move the actuator 65 to actuate the canister valve 67.

It is contemplated that the apparatus of the present disclosure can comprise the features described herein that permit the operator to deliver a fluid from a canister to (and through) the curette head and/or the apparatus can comprise the features described herein that permit the operator to generate negative pressure in the body 980 of the apparatus in order to draw a fluid and/or debris through the curette head and into a chamber in the apparatus.

Advantageously, the elongated member 2000 can be operated with a single hand both to deliver fluid to a treatment site and to remove fluid and debris (e.g., via the suction force created in the chamber 64) from the treatment site. In any embodiment, the elongated member 2000 can be employed as a single-use instrument, which is subsequently discarded.

In any embodiment, the apparatus may include an actuator capable of generating a high-speed, high-pressure pulse that is both controllable and highly predictable. The apparatus may be combined with a servo-controller receiving inputs from one or more sensors. The one or more sensors may be disposed in the curette head and/or in the handle. Further, the apparatus may adjust or tailor the pressure profile of a transfer in real-time, during the course of the transfer, responsive to sensed physical properties of a wound site. For example, the one or more sensor of the apparatus may be able to distinguish living tissue from non-living tissue debris.

The jet injector may include sensors that detect respective physical properties of the treatment site. The physical properties can be used to servo control the jet injector and tailor the injection pressure, and, therefore, the depth of penetration of fluid into for a particular region. For instance, when the apparatus is used on a healing portion of a wound site, the sensor detects the viable tissue, and the controller uses the properties of the viable tissue and consequently reduces the injection pressure. The injection pressure can be adjusted, for example, by controlling the electrical input signal applied to the injector and/or the current pulse rise time and/or duration. When used on dead tissue and/or tissue debris, the controller may increase the injection pressure. The injection pressure may be adjusted depending on location of the liquid transfer, for example, the live tissue versus dead tissue. Moreover, the transfer pressure may be varied over time.

In certain embodiments, the needle-free injector generates a jet pressure and transfers high pressure liquid that can remove the dead tissue and/or tissue debris from the wound site. The peak liquid pressure may be as low as 1 Kilo Pascal. In some embodiments up to 100 Kilo Pascal of relative water pressure may be applied.

Jets of less than 500 microns are generally employed. In certain embodiments, very fine jets may be employed. For example, jets having diameter of less than five micro meters may be employed. In one embodiment, the jet injectors may have a diameter of less than 200 micrometers.

In any embodiment, the apparatus is servo-controlled to transfer the fluid and control the pressure of the fluid in the vicinity of live tissue and dead tissue and/or tissue debris. Due to the high level of control offered by the apparatus, the amount of fluid transferred into the wound site may be significantly reduced, relative to standard debridement or curettage procedures. In some embodiments, a liquid or foam having appropriate cleaning agents may be jet injected into the wound site to facilitate the removal of tissue or tissue debris. Additional substances may be included in the liquid injected into the wound site. For example, antimicrobial and/or emollient substances may be employed.

In any embodiment, the apparatus is operatively coupled to a fluid source (e.g., an aqueous rinse fluid) and a controllable electromagnetic actuator in communication with the fluid source. The controllable electromagnetic actuator may include a stationary magnet assembly providing a magnetic field; and a coil assembly. The coil assembly receives an electrical input and generates in response a force corresponding to the received input. The force results from interaction of an electrical current within the coil assembly and the magnetic field and causes the transfer of the fluid between the fluid source and the wound site. The apparatus can include a sensor that senses physical properties of the transfer site and causes the servo-controller to generate the electrical input responsive to the sensed physical property. In order to measure the physical properties, ranging techniques using acoustic waves or laser beams may be applied to delineate areas of live tissue and dead tissue (and/or tissue debris). Acoustic waves may be used in combination with a fluid jet, where the pressure of the fluid jet applies a force to the target tissue to perturb the target tissue and the acoustic waves are used to sense the deformation or displacement of the tissue in response to the perturbation. The acoustic signal can propagate through the fluid jet and may include a sinusoidal and/or stochastic signal.

Example embodiments may include a Lorentz Force actuator to provide reversibility for the apparatus. In such embodiments, a fluid can be pumped into the wound site to lubricate the curette head blades and/or to loosen or remove tissue or tissue debris from the blades or the wound site. The removed material may then be removed from the blades or wound site via the fourth and/or fifth openings in the curette head. The Lorentz force actuator exerts a force. The force can be used for needle-free transfer of the substance between the fluid source and the wound site. The injector can determine changes in response to the forces and determine individual target area properties based on the response to the forces. In certain embodiments, stochastic (random) or pseudo-random information can be employed to create a model (e.g., non-parametric model) of tissue properties.

Example embodiments may include a piezoelectric actuator to provide high frequency pulses of the fluid jet. The apparatus may have a minimum bandwidth of approximately 10 Hz (Hertz). In certain embodiments the apparatus may have a bandwidth of 50 Hz, 100 Hz, or 100 KHz.

Certain embodiments may include sensors and related technologies for analyzing materials (e.g., tissue, tissue debris, and/or wound exudate) removed from the wound site for diagnostic indicators such as indicators of healing (e.g., matrix metalloproteinases), for example. The analyzed results may be used to distinguish between healthy and unhealthy tissue in the wound site. In some embodiments, the liquid transferred into the wound site may include biomarkers for verifying wound tissue health and for detecting possible health issues. In some embodiments, the same liquid-containing biomarkers may be used in cleaning and tissue/debris removal.

The apparatus may transfer as much as 10 microliters of fluid, e.g., liquid, per second. The liquid may be transferred at a peak pressure of 1 kilo Pascal to loosen food. In certain embodiments, an average pressure of up to 100 Mega Pascal may be used to loosen dead tissue and/or tissue debris. The apparatus may operate at a velocity as low as 1 meter per second (m/s). In some embodiments, velocities as high as 10 m/s or higher (e.g., 2/3 of the speed of sound) may be used.

Pulses can be used to dynamically control (e.g., servo-control) the magnitude, direction and duration of the force during the course of an actuation cycle. In any embodiment, the apparatus may include an actuator capable of generating a high-speed, high-pressure pulse that is both controllable and highly predictable. The apparatus also includes the ability to pulse shape to use different waveforms for each cycle. In any embodiment, fine sinusoidal pulses may be employed to approximate a square wave. For example, a sinusoidal pulse having a bandwidth of 10 microseconds can be used.

The apparatus may be coupled to multiple fluid sources (e.g., reservoirs). In one example embodiment, the apparatus includes two reservoirs arranged such that one reservoir includes a larger amount of fluid compared to the other reservoir. At each cycle, a square wave is used to control the direction and duration of the force. At each cycle a certain amount of fluid is transferred from the second reservoir into the wound site. At the end of the cycle, a limited amount of liquid is transferred from the first reservoir into the second reservoir.

An exemplary apparatus that comprises a fluid injector (e.g., a jet injector capable of high-speed, high-pressure fluid injection) operatively coupled to a fluid source, an actuator (e.g., a Lorentz force actuator), and optional sensors is described in U.S. Patent Application Publication No. 2011/0311939.

Thus, in any embodiment of the apparatus of the present disclosure, the apparatus optionally can comprise a jet injector (e.g., a jet injector as described in U.S. Patent Application Publication No. 2011/0311939). Furthermore, the jet injector may be operatively coupled to a Lorentz force actuator and/or a sensor, as described in U.S. Patent Application Publication No. 2011/0311939, for example.

FIG. 21 is a partially-exploded schematic block diagram showing the first end 3001 of an apparatus 3000 comprising a jet injector that may be used in conjunction with an elongated member according to the present disclosure. The apparatus may be used to eject a fluid through a first hollow channel 142 of a curette head 100 (shown in cross-section in FIG. 21) in order to facilitate debridement of a wound site (not shown).

The apparatus 3000 may include a nozzle 81 to convey a substance (e.g., a sterile liquid) into the curette head 100. Namely, substance ejected from the nozzle 81 forms a jet, the force of the jet determining the depth of penetration of the liquid into the wound site. The nozzle 81 may contain a flat surface, such as the head 82 and an orifice 70. It is the inner diameter of the orifice 70 that controls the diameter of the transferred stream. Additionally, the length of an aperture or tube 71, defining the orifice 70 also controls the transfer (e.g., injection) pressure.

The nozzle 81 can be coupled to a reservoir 79 for temporarily storing the transferred substance. Reservoir 79 may be a reservoir of a syringe or ampoule. Beneficially, a pressure is selectively applied to the reservoir 79 using a controllable actuator. A specially-designed electromagnetic actuator 84 is configured to generate a high-pressure pulse having a rapid rise time (e.g., less than 1 millisecond). The actuator 84 can be used in devices that rely on high-pressure actuators to inject a formulation (e.g., a liquid material) into a wound site. The actuator is dynamically controllable, allowing for adjustments to the pressure-versus-time during actuation. Actuation involves movement of a freely suspended coil within a gap, rather than the sudden release of a spring or the discharge of a gas. Actuation of the freely moving coil in the manner described herein results in quiet operation, which is an important feature as it contributes to reducing pain and anxiety during treatment of the wound site.

The electromagnetic actuator 84 is configured to provide a linear force applied to the plunger 85 to achieve transfer of the substance. Transfer of the force can be accomplished with a force-transfer member 76, such as a rigid rod slidably coupled through a bearing 77. The rod may be secured at either end such that movement of the actuator in either direction also moves the plunger 85. The bearing restricts radial movement of the rod 76, while allowing axial movement.

In any embodiment, the actuator 84 is a Lorentz force actuator that includes a stationary component, such as a magnet assembly 73, and a moveable component, such as a coil assembly 72. A force produced within the coil assembly 72 can be applied to the plunger 85 either directly or indirectly through the rod 76 to achieve transfer of the substance.

In some embodiments, apparatus 3000 may not include a separate bearing 77. Rather, an interior surface of the handle 900 provides a bearing for the coil assembly 72 allowing axial movement while inhibiting radial movement.

A power source 74 provides an electrical input to the coil assembly 72 of the actuator 84. An electrical current applied to the coil assembly 72 in the presence of a magnetic field provided by the magnet assembly 73 will result in a generation of a mechanical force capable of moving the coil assembly 72 and exerting work on the plunger 85 of the syringe 86. The electromagnetic actuator is an efficient force transducer supporting its portability.

A controller 75 is electrically coupled between the power source 74 and the actuator 84, such that the controller 75 can selectively apply, withdraw and otherwise adjust the electrical input signal provided by the power source 74 to the actuator 84. The controller 75 can be a simple switch that is operable by a local interface. For example, a button provided on the handle 900 may be manipulated by a user, selectively applying and removing an electrical input from the power source 74 to the actuator 84. In some embodiments, the controller 75 includes control elements, such as electrical circuits, that are adapted to selectively apply electrical power from the power source 74 to the actuator 84, the electrical input being shaped by the selected application. Thus, for embodiments in which the power source 74 is a simple battery providing a substantially constant or direct current (D.C.) value, the electrical input can be shaped by the controller to provide a different or even time varying electrical value. In some embodiments, the controller 75 includes an on-board microprocessor, or alternatively an interconnected processor or personal computer providing multifunction capabilities. A power amplifier (not shown) may be included in the controller 75 or, alternatively or in addition, in power source 74.

In some embodiments the controller receives inputs from one or more sensors adapted to sense a respective physical property. For example, the apparatus 3000 includes a transducer, such as a position sensor 83B used to indicate location of an object's coordinates (e.g., the coil's position) with respect to a selected reference. Similarly, a displacement may be used to indicate movement from one position to another for a specific distance. Beneficially, the sensed parameter can be used as an indication of the plunger's position and therefore an indication of dose. In some embodiments, a proximity sensor may also be used to indicate when a part of the device, such as the coil, has reached a critical distance. This may be accomplished by sensing the position of the plunger 85, the force-transfer member 76, or the coil assembly 72 of the electromagnetic actuator 84. For example, an optical sensor such as an optical encoder can be used to count turns of the coil to determine the coil's position. Other types of sensors suitable for measuring position or displacement include inductive transducers, resistive sliding-contact transducers, photodiodes, and linear-variable-displacement-transformers (LVDT).

Other sensors, such as a force transducer 83A can be used to sense the force applied to the plunger 85 by the actuator 84. As shown, a force transducer 83A can be positioned between the distal end of the coil assembly and the force transfer member 76, the transducer 83A sensing force applied by the actuator 84 onto the force-transfer member 76. As this member 76 is rigid, the force is directly transferred to the plunger 85. The force tends to move the plunger 85 resulting in the generation of a corresponding pressure within the reservoir 79. A positive force pushing the plunger 85 into the reservoir 79 creates a positive pressure tending to force a substance within the reservoir 79 out through the nozzle 81. A negative force pulling the plunger 85 proximally away from the nozzle 81 creates a negative pressure or vacuum tending to suck a substance from outside the device through the nozzle 81 into the reservoir 79. The substance may also be obtained from another reservoir or ampoule, the negative pressure being used to pre-fill the reservoir 79 with the substance. In some embodiments, a pressure transducer (not shown) can also be provided to directly sense the pressure applied to a substance within the chamber or reservoir 79. In addition, the position sensor 83B may sense the position of the coil which may be used to indirectly measure the pressure within the reservoir 79. An electrical sensor 83C may also be provided to sense an electrical input provided to the actuator 84. The electrical sensor may sense one or more of coil voltage and coil current. Other sensors are also contemplated, as described in U.S. Patent Application Publication No. US 2011/0311939 A1.

Beneficially, the substance (e.g., a sterile liquid) delivered from the jet injector to the wound site can facilitate disintegration of nonviable tissue and/or wound debris at the treatment site, thereby making it more susceptible to removal by one or more blade of the curette head and making it more susceptible to be transported through the second hollow channel and/or second conduit of the apparatus of the present disclosure.

In another aspect, the present disclosure further provides a kit. The kit comprises the any embodiment of the curette head according to the present disclosure. In any embodiment, the kit optionally may comprise a plurality of curette heads. In any embodiment, the kit further comprises a handle configured to be operatively attached to at least one of the curette heads. In any embodiment, "operatively attaching the handle to the curette head" optionally comprises providing a first fluid pathway through the handle to the first hollow channel. In addition, in any embodiment, "operatively attaching the handle to the curette head" optionally further comprises providing a second fluid pathway through the handle to the second hollow channel.

Curette heads and apparatuses of the present disclosure can be used in various exemplary methods (e.g., a method to treat a wound site). In a first aspect, the method comprises operatively connecting, to a source of fluid (e.g., a liquid such as water, a saline solution, a buffered saline solution), the first opening of the curette head of any apparatus according to the present disclosure; placing at least two blades of the curette head of the apparatus into contact with tissue and/or tissue debris proximate a treatment site (e.g., a wound site); and while in contact with the tissue and/or tissue debris, moving the blades laterally relative to the wound site. In any embodiment of the first aspect, the method further comprises operatively connecting the third opening of the curette head to a source of negative pressure. In a second aspect, the method comprises operatively connecting, to a source of negative pressure, the third opening of the curette head of any apparatus according to the present disclosure; placing at least two blades of the curette head of the apparatus into contact with tissue and/or tissue debris proximate a treatment site (e.g., a wound site); and while in contact with the tissue and/or tissue debris, moving the blades laterally relative to the wound site. In any embodiment of the second aspect, the method further comprises operatively connecting the first opening to a source of a fluid; and flowing the fluid through the at least one second opening of the curette head.

Advantageously, any embodiment of the curette head or apparatus of the present disclosure enables the user to deliver a fluid (e.g., a rinsing and/or cleansing fluid) to a location immediately proximate the debridement edge of a first blade (e.g., the first blade disclosed herein) and, optionally can remove the delivered fluid directly through one or more additional blade (e.g., the second blade and/or the third blade disclosed herein) located proximate the first blade. This can reduce the amount of liquid needed to treat (e.g., debride) a treatment site and/or it can reduce or eliminate run-off of excess liquid delivered to a treatment site.

The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

All headings are for the convenience of the reader and should not be used to limit the meaning of the text that follows the heading, unless so specified.

The invention illustratively described herein suitably may be practiced in the absence of any element(s) not specifically disclosed herein. Thus, for example, in each instance herein any of the terms "comprising", "consisting essentially of", and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

## Claims

1. A curette head (100), comprising:
a base (109);
a plurality of blades (110, 120, 130) extending from the base;
wherein each blade of the plurality of blades comprises a debridement edge (118, 128, 138) and a longitudinal dimension extending from the base to the debridement edge;
wherein the debridement edge of each blade of the plurality of blades is defined by a first major surface (151) and a second major surface (152) of each respective blade of the plurality of blades;
wherein the plurality of blades comprises a first blade (110); and
at least one first hollow channel (142) extending through the first blade;
wherein the at least one first hollow channel extends along the first blade longitudinal dimension from a first opening (191) in the base, or proximate the base, to at least one second opening (192) that is integrated in the debridement edge of the first blade or that is disposed in the first major surface of the first blade proximate the debridement edge of the first blade.

2. The curette head of claim 1, wherein the plurality of blades further comprises a second blade, wherein at least one second hollow channel extends through the second blade, wherein the at least one second hollow channel extends along the second blade longitudinal dimension from a third opening in the base or proximate the base to at least one fourth opening that is integrated in the debridement edge of the second blade or that is disposed in the first surface of the second blade proximate the debridement edge of the second blade.

3. The curette head of claim 1 or claim 2, wherein the plurality of blades further comprises a third blade, wherein at least a portion of the second hollow channel extends through the third blade, wherein the portion of the second hollow channel extends along the third blade longitudinal dimension from an opening in the base or proximate the base to at least one fifth opening that is integrated in the debridement edge of the third blade or that is disposed in the first major surface of the third blade proximate the debridement edge of the third blade.

4. The curette head of any one of the preceding claims, wherein the first blade, second blade, or third blade substantially comprises a polymeric material.

5. The curette head of any one of the preceding claims, wherein the curette head comprises a unitary body that includes the base, at least one of the plurality of blades, and at least one of the hollow channels.

6. A kit, comprising a curette head of any one of the preceding claims.

7. The kit of claim 6, further comprising a handle configured to be operatively attached to at least one of the curette heads.

8. An apparatus, comprising:
an elongate member (1000) having a first end (1001) and a second end (1002);
the curette head (100) of any of claims 1 to 7; and
a handle (900) disposed at the second end, the handle attached to the curette head (100) and comprising a first conduit (954) extending therethrough, wherein the first conduit is in fluidic communication with the first hollow channel (142).

9. The apparatus of claim 8, wherein the first blade comprises a plurality of second openings.

10. The apparatus of claim 8 or claim 9:
wherein the handle further comprises a second conduit extending therethrough;
wherein the plurality of blades further comprises a second blade;
wherein at least one second hollow channel extends through the second blade;
wherein the at least one second hollow channel extends along the second blade longitudinal dimension from a third opening in the base, or proximate the base, to at least one fourth opening that is integrated in the debridement edge of the second blade or that is disposed in the first major surface of the second blade proximate the debridement edge of the second blade;
wherein the second conduit is in fluidic communication with the second hollow channel.

11. The apparatus of any one of claims 8 through 10:
wherein the handle further comprises a second conduit extending therethrough;
wherein the plurality of blades further comprises a third blade;
wherein at least a portion of the second hollow channel extends through the third blade;
wherein the portion of the second hollow channel extends along the third blade longitudinal dimension from an opening in the base, or proximate the base, to at least one fifth opening that is integrated in the debridement edge of the third blade or that is disposed in the first major surface of the third blade proximate the debridement edge of the third blade;
wherein the second conduit is in fluidic communication with the second hollow channel.

12. The apparatus of any one of claims 8 through 11, wherein the first conduit and/or the first hollow channel is fluidically connected to a source of fluid.

13. The apparatus of claim 12, further comprising a jet injector fluidically connected to the source of fluid.

14. The apparatus of any one of claims 10 through 13, wherein the second hollow channel is fluidically connected to a source of negative pressure.

## Patentansprüche

1. Kürettenkopf (100), umfassend:
eine Basis (109);
mehrere Klingen (110, 120, 130), die sich von der Basis aus erstrecken;
wobei jede Klinge der mehreren Klingen eine Wundausscheidungs-Kante (118, 128, 138) und eine Längsabmessung, die sich von der Basis zu der Wundausscheidungs-Kante erstreckt, umfasst;
wobei die Wundausscheidungs-Kante jeder Klinge der mehreren Klingen durch eine erste Hauptfläche (151) und eine zweite Hauptfläche (152) jeder jeweiligen Klinge der mehreren Klingen bestimmt ist;
wobei die mehreren Klingen eine erste Klinge (110) und mindestens einen ersten hohlen Kanal (142), der sich durch die erste Klinge erstreckt, umfassen;
wobei der mindestens eine erste hohle Kanal sich entlang der Längsabmessung der ersten Klinge von einer ersten Öffnung (191) in der Basis oder in der Nähe der Basis zu mindestens einer zweiten Öffnung (192) erstreckt, die in die Wundausscheidungs-Kante der ersten Klinge integriert ist oder die in der ersten Hauptfläche der ersten Klinge in der Nähe der Wundausscheidungs-Kante der ersten Klinge angeordnet ist.

2. Kürettenkopf nach Anspruch 1, wobei die mehreren Klingen ferner eine zweite Klinge umfassen, wobei mindestens ein zweiter hohler Kanal sich durch die zweite Klinge erstreckt, wobei der mindestens eine zweite hohle Kanal sich entlang der Längsabmessung der zweiten Klinge von einer dritten Öffnung in der Basis oder in der Nähe der Basis zu mindestens einer vierten Öffnung erstreckt, die in die Wundausscheidungs-Kante der zweiten Klinge integriert ist oder die in der ersten Oberfläche der zweiten Klinge in der Nähe der Wundausscheidungs-Kante der zweiten Klinge angeordnet ist.

3. Kürettenkopf nach Anspruch 1 oder Anspruch 2, wobei die mehreren Klingen ferner eine dritte Klinge umfassen, wobei wenigstens ein Abschnitt des zweiten hohlen Kanals sich durch die dritte Klinge erstreckt, wobei der Abschnitt des zweiten hohlen Kanals sich entlang der Längsabmessung der dritten Klinge von einer Öffnung in der Basis oder in der Nähe der Basis zu mindestens einer fünften Öffnung erstreckt, die in die Wundausscheidungs-Kante der dritten Klinge integriert ist oder die in der ersten Hauptoberfläche der dritten Klinge in der Nähe der Wundausscheidungs-Kante der dritten Klinge angeordnet ist.

4. Kürettenkopf nach einem der vorstehenden Ansprüche, wobei die erste Klinge, die zweite Klinge oder die dritte Klinge im Wesentlichen ein Polymermaterial umfasst.

5. Kürettenkopf nach einem der vorstehenden Ansprüche, wobei der Kürettenkopf einen einstückigen Körper umfasst, der die Basis, mindestens eine der mehreren Klingen und mindestens einen der hohlen Kanäle umfasst.

6. Kit, umfassend einen Kürettenkopf nach einem der vorstehenden Ansprüche.

7. Kit nach Anspruch 6, ferner umfassend einen Griff, der dazu konfiguriert ist, wirkend an mindestens einem der Kürettenköpfe befestigt zu werden.

8. Vorrichtung, umfassend:
ein langgestrecktes Element (1000) mit einem ersten Ende (1001) und einem zweiten Ende (1002);
den Kürettenkopf (100) nach einem der Ansprüche 1 bis 7; und
einen Griff (900), der an dem zweiten Ende angeordnet ist, wobei der Griff an dem Kürettenkopf (100) befestigt ist und eine erste Leitung (954), die sich durch ihn hindurch erstreckt, umfasst, wobei die erste Leitung in Fluidaustausch mit dem ersten hohlen Kanal (142) steht.

9. Vorrichtung nach Anspruch 8, wobei die erste Klinge mehrere zweite Öffnungen umfasst.

10. Vorrichtung nach Anspruch 8 oder Anspruch 9:
wobei der Griff ferner eine zweite Leitung umfasst, die sich durch ihn hindurch erstreckt;
wobei die mehreren Klingen ferner eine zweite Klinge umfassen;
wobei mindestens ein zweiter hohler Kanal sich durch die zweite Klinge erstreckt;
wobei der mindestens eine zweite hohle Kanal sich entlang der Längsabmessung der zweiten Klinge von einer dritten Öffnung in der Basis oder in der Nähe der Basis zu mindestens einer vierten Öffnung erstreckt, die in die Wundausscheidungs-Kante der zweiten Klinge integriert ist oder die in der ersten Hauptoberfläche der zweiten Klinge in der Nähe der Wundausscheidungs-Kante der zweiten Klinge angeordnet ist;
wobei die zweite Leitung in Fluidaustausch mit dem zweiten hohlen Kanal steht.

11. Vorrichtung nach einem der Ansprüche 8 bis 10:
wobei der Griff ferner eine zweite Leitung umfasst, die sich durch ihn hindurch erstreckt;
wobei die mehreren Klingen ferner eine dritte Klinge umfassen;
wobei wenigstens ein Abschnitt des zweiten hohlen Kanals sich durch die dritte Klinge erstreckt;
wobei der Abschnitt des zweiten hohlen Kanals sich entlang der Längsabmessung der dritten Klinge von einer Öffnung in der Basis oder in der Nähe der Basis zu mindestens einer fünften Öffnung erstreckt, die in die Wundausscheidungs-Kante der dritten Klinge integriert ist oder die in der ersten Hauptoberfläche der dritten Klinge in der Nähe der Wundausscheidungs-Kante der dritten Klinge angeordnet ist;
wobei die zweite Leitung in Fluidaustausch mit dem zweiten hohlen Kanal steht.

12. Vorrichtung nach einem der Ansprüche 8 bis 11, wobei die erste Leitung und/oder der erste hohle Kanal fluidisch mit einer Fluidquelle verbunden sind.

13. Vorrichtung nach Anspruch 12, ferner umfassend einen Jet-Injektor, der fluidisch mit der Fluidquelle verbunden ist.

14. Vorrichtung nach einem der Ansprüche 10 bis 13, wobei der zweite hohle Kanal fluidisch mit einer Unterdruckquelle verbunden ist.

## Revendications

1. Tête de curette (100), comprenant :
une base (109) ;
une pluralité de lames (110, 120, 130) s'étendant à partir de la base ;
dans laquelle chaque lame de la pluralité de lames comprend un bord de débridement (118, 128, 138) et une dimension longitudinale s'étendant de la base au bord de débridement ;
dans laquelle le bord de débridement de chaque lame de la pluralité de lames est défini par une première surface principale (151) et une deuxième surface principale (152) de chaque lame respective de la pluralité de lames ;
dans laquelle la pluralité de lames comprend une première lame (110) ; et au moins un premier canal creux (142) s'étendant à travers la première lame ;
dans laquelle ledit au moins un premier canal creux s'étend le long de la dimension longitudinale de première lame d'une première ouverture (191) dans la base, ou à proximité de la base, à au moins une deuxième ouverture (192) qui est intégrée dans le bord de débridement de la première lame ou qui est disposée dans la première surface principale de la première lame à proximité du bord de débridement de la première lame.

2. Tête de curette selon la revendication 1, dans laquelle la pluralité de lames comprend en outre une deuxième lame, dans laquelle au moins un deuxième canal creux s'étend à travers la deuxième lame, dans laquelle ledit au moins un deuxième canal creux s'étend le long de la dimension longitudinale de deuxième lame d'une troisième ouverture dans la base ou à proximité de la base à au moins une quatrième ouverture qui est intégrée dans le bord de débridement de la deuxième lame ou qui est disposée dans la première surface de la deuxième lame à proximité du bord de débridement de la deuxième lame.

3. Tête de curette selon la revendication 1 ou la revendication 2, dans laquelle la pluralité de lames comprend en outre une troisième lame, dans laquelle au moins une partie du deuxième canal creux s'étend à travers la troisième lame, dans laquelle la partie du deuxième canal creux s'étend le long de la dimension longitudinale de troisième lame d'une ouverture dans la base ou à proximité de la base à au moins une cinquième ouverture qui est intégrée dans le bord de débridement de la troisième lame ou qui est disposée dans la première surface principale de la troisième lame à proximité du bord de débridement de la troisième lame.

4. Tête de curette selon l'une quelconque des revendications précédentes, dans laquelle la première lame, la deuxième lame ou la troisième lame comprend essentiellement un matériau polymère.

5. Tête de curette selon l'une quelconque des revendications précédentes, dans laquelle la tête de curette comprend un corps d'un seul tenant qui inclut la base, au moins une parmi la pluralité de lames, et au moins un des canaux creux.

6. Trousse, comprenant une tête de curette selon l'une quelconque des revendications précédentes.

7. Trousse selon la revendication 6, comprenant en outre un manche conçu pour être fixé opérationnellement à au moins une des têtes de curette.

8. Appareil comprenant :
un élément allongé (1000) comportant une première extrémité (1001) et une deuxième extrémité (1002) ;
la tête de curette (100) selon l'une quelconque des revendications 1 à 7 ; et
un manche (900) disposé au niveau de la deuxième extrémité, le manche fixé à la tête de curette (100) et comprenant un premier conduit (954) s'étendant à travers celui-ci, dans lequel le premier conduit est en communication fluidique avec le premier canal creux (142).

9. Appareil selon la revendication 8, dans lequel la première lame comprend une pluralité de deuxièmes ouvertures.

10. Appareil selon la revendication 8 ou la revendication 9 :
dans lequel le manche comprend en outre un deuxième conduit s'étendant à travers celui-ci ;
dans lequel la pluralité de lames comprend en outre une deuxième lame ;
dans lequel au moins un deuxième canal creux s'étend à travers la deuxième lame ;
dans lequel ledit au moins un deuxième canal creux s'étend le long de la dimension longitudinale de deuxième lame d'une troisième ouverture dans la base, ou à proximité de la base, à au moins une quatrième ouverture qui est intégrée dans le bord de débridement de la deuxième lame ou qui est disposée dans la première surface principale de la deuxième lame à proximité du bord de débridement de la deuxième lame ;
dans lequel le deuxième conduit est en communication fluidique avec le deuxième canal creux.

11. Appareil selon l'une quelconque des revendications 8 à 10 :
dans lequel le manche comprend en outre un deuxième conduit s'étendant à travers celui-ci ;
dans lequel la pluralité de lames comprend en outre une troisième lame ;
dans lequel au moins une partie du deuxième canal creux s'étend à travers la troisième lame ;
dans lequel la partie du deuxième canal creux s'étend le long de la dimension longitudinale de troisième lame d'une ouverture dans la base, ou à proximité de la base, à au moins une cinquième ouverture qui est intégrée dans le bord de débridement de la troisième lame ou qui est disposée dans la première surface principale de la troisième lame à proximité du bord de débridement de la troisième lame ;
dans lequel le deuxième conduit est en communication fluidique avec le deuxième canal creux.

12. Appareil selon l'une quelconque des revendications 8 à 11, dans lequel le premier conduit et/ou le premier canal creux est raccordé fluidiquement à une source de fluide.

13. Appareil selon la revendication 12, comprenant en outre un injecteur sans aiguille raccordé fluidiquement à la source de fluide.

14. Appareil selon l'une quelconque des revendications 10 à 13, dans lequel le deuxième canal creux est raccordé fluidiquement à une source de pression négative.
